# EUROPEAN PATENT APPLICATION

(11) **EP 2 258 265 A2**
(43) Date of publication of application: **08.12.2010**
(21) Application number: 09012299.5
(22) Date of filing: 28.09.2009
(51) Int. Cl.: A61B 5/107

(54) **Human body measurement system and information provision method using the same**

(30) Priority: 03.06.2009 KR 20090048859
(71) Applicant: MINIMEDREAM Co., Ltd., Seoul-si (KR)
(72) Inventor: Kim, Chang Ho, Seoul-si (KR)
(74) Representative: Cabinet Plasseraud

(57) **Abstract**

Disclosed herein are a human body measurement system and an information provision method using the human body measurement system. The human body measurement system is a system into which a human body measurement apparatus and a measurement unit are incorporated. The human body measurement system includes a photographing unit (210) configured to include a plurality of cameras and a hub and send images, captured by the cameras, to a PC of an integrated controller through the hub, a weight scale (220) configured to measure body weight of a subject of measurement, and a body composition analyzer (230) configured to measure body composition of the subject of measurement.

## Description

### BACKGROUND OF THE INVENTION

### 1. Field of the Invention

The present invention relates to a three-dimensional (3D) scan system using 3D modeling technology, which is capable of measuring and analyzing the shape, weight and composition of a human body using a photographing unit and allowing checking of health-related conditions or acquiring a variety of health information and human body measurement information using analysis results, and an information provision method using the system.

### 2. Description of the Related Art

3D modeling technology can extract 3D data through actual photographing, unlike existing object scanning technology.

Hitherto, there have been cases where body photos, such as simple full-length photos or facial photos, are captured and used for medical and clothing purposes. However, these cases are disadvantageous in that it is difficult to implement 3D shape models because they chiefly use frontal photos.

Conventional technology for capturing the shape of an object and/or a human includes a method of scanning shape information using an active sensor based on laser or pattern light and extracting the texture of scanned meshes from captured color images. This method is chiefly limited to static objects, and is used for the creation of high-quality models. However, this method has disadvantages in that the restoration of a static object requires an excessively long period of time and the restoration of a dynamic object does not guarantee the restoration of a real 3D image.

Meanwhile, although many apparatuses for analyzing body composition have been disclosed, a variety of visual data is not effectively represented so that it can be viewed at a glance, with the result that the length of the period required for the analysis and the accuracy of the analysis are limited.

### SUMMARY OF THE INVENTION

Accordingly, the present invention has been made keeping in mind the above problems occurring in the prior art, and an object of the present invention is to make the speed of processing of captured photo data fast and make a variety of types of 3D modeling editing possible.

Another object of the present invention is to enable the measurement of body composition and human body of a subject to be performed at the same time, so that the uncertainty of data provided by a subject of measurement can be eliminated, thereby increasing the accuracy of measurement and analysis results.

Still another object of the present invention is to enable the measurement of a human body and the capturing of photos to be performed simultaneously, thereby allowing desired data to be acquired within a significantly short period of time, as well as to increase the accuracy of analysis.

In order to accomplish the above object, the present invention provides a human body measurement system into which a human body measurement apparatus and a measurement unit are incorporated, including a photographing unit configured to include a plurality of cameras and a hub and send images, captured by the cameras, to a PC of an integrated controller through the hub; a weight scale configured to measure body weight of a subject of measurement; and a body composition analyzer configured to measure body composition of the subject of measurement.

Preferably, the image data captured by the photographing unit is three-dimensionally modeled to provide the measurement of length and volume of each portion of a human body, 3D simulation is provided based on the measured data, and information about exercise and dietary prescriptions is provided based on the measurement of the body weight using the weight scale and the analysis of the body composition using the body composition analyzer; wherein the simulation and the information are provided simultaneously.

Preferably, the body weight is measured using the weight scale at the same time that photographing is performed using the photographing unit, height is measured using the captured data, and the measured data is automatically input to the body composition analyzer and used for analysis of the body composition data measured by the body composition analyzer.

Preferably, a 3D shape model is formed by approximating shape information using polygons through extraction of colored corresponding points of multiview images captured by the photographing unit, connecting polygons of each section to each other and then creating a complete mesh structure.

Preferably, the human body measurement system further includes a system control unit for testing and maintaining the system and controlling the photographing unit, the weight scale, the body composition analyzer, an internal monitor, an illuminator, a speaker and a microphone through a Personal Computer (PC); an integrated controller for analyzing and storing acquired images, body weight data and body composition data through the system control unit; an operating desk for connecting to the integrated controller over a network and remotely managing and operating the cameras and the analyzer; and a power supply for supplying power to the system.

Preferably, the measurement apparatus further includes an internal monitor for enabling the subject of measurement to monitor the status of progress.

Preferably, the operating desk includes a Personal Computer (PC) for referring to and managing measured data, performing simulation analysis and offering prescriptions, and a communication microphone and speaker for transmitting information about a position of measurement and the status of progress to the subject of measurement.

Preferably, the system control unit includes a camera control unit for controlling calibration and photographing processes; a human body measuring unit for performing 3D modeling and performing measurement based on captured images of a human body of the subject of measurement; a body composition analysis unit for analyzing body composition and offering prescriptions on the basis of data measured by the body composition analysis unit; a body weight analysis unit for analyzing and managing body weight based on data measured by the weight scale; a monitor control unit for controlling the internal monitor; and a voice communication unit for managing the speaker and the microphone.

Additionally, the present invention provides an information provision method using the human body measurement system, including the steps of entering basic information of a subject of measurement and performing ID authentication login; an operator at the operating desk guiding the subject of measurement through a measurement method and checking whether the subject of measurement has entered the scan apparatus; the operator guiding the subject of measurement through positioning of a human body; returning to the guiding step if a position of the human body of the subject of measurement is inappropriate, and performing photographing using the cameras if a position of the human body is appropriate; guiding the subject of measurement through a procedure of measuring body weight and body composition through the internal monitor; measuring the body weight and body composition of the subject of measurement using a weight scale and a body composition analyzer; after the measurement, the subject of measurement exiting the scan apparatus; and storing measured data about the subject of measurement in the integrated controller, analyzing the measured data, and offering prescriptions.

Preferably, the step of storing measured data, analyzing the measured data and offering prescriptions comprises the step of storing raw data of images of the subject of measurement acquired by the cameras in a DB in the form of data about the subject of measurement regarding 3D modeling, human body measurement and plastic surgery via voxel data (3D pixel data) and mesh data, and storing data about the analysis of the body composition analysis, the prescriptions and a comprehensive opinion based on the raw data of the body composition and the body weight in the DB as well.

Preferably, the information provision method further includes, after the step of storing measured data, analyzing the measured data and offering prescriptions, the step of outputting data about the analysis and the prescription.

### BRIEF DESCRIPTION OF THE DRAWINGS

The above and other objects, features and advantages of the present invention will be more clearly understood from the following detailed description taken in conjunction with the accompanying drawings, in which:
FIG. 1 is a diagram showing the hardware construction of a scan system according to the present invention;
FIG. 2 is a block diagram showing the construction of the scan system according to the present invention;
FIG. 3 is a diagram showing the software construction of the scan system according to the present invention;
FIG. 4 is a block diagram showing the operation of the scan system according to the present invention;
FIG. 5 is a flowchart showing the operation of the scan system according to the present invention;
FIG. 6 is a diagram showing the construction of a main output screen of the scan system according to the present invention;
FIG. 7 is a diagram showing the construction of a contour line screen of the scan system according to the present invention;
FIG. 8 is a diagram showing the construction of a human body measurement screen of the scan system according to the present invention;
FIG. 9 is a diagram showing the construction of a simulation input screen of the scan system according to the present invention;
FIG. 10 is a diagram showing the construction of a human body measurement screen of the scan system according to the present invention;
FIG. 11 is a diagram showing the construction of an exercise prescription screen of the scan system according to the present invention;
FIG. 12 is a diagram showing the construction of a dietary prescription screen of the scan system according to the present invention; and
FIG. 13 is a diagram showing an embodiment of a 3D modeling method according to the present invention.

### DESCRIPTION OF THE PREFERRED EMBODIMENTS

Preferred embodiments of the present invention will be described in detail below with reference to the following drawings.

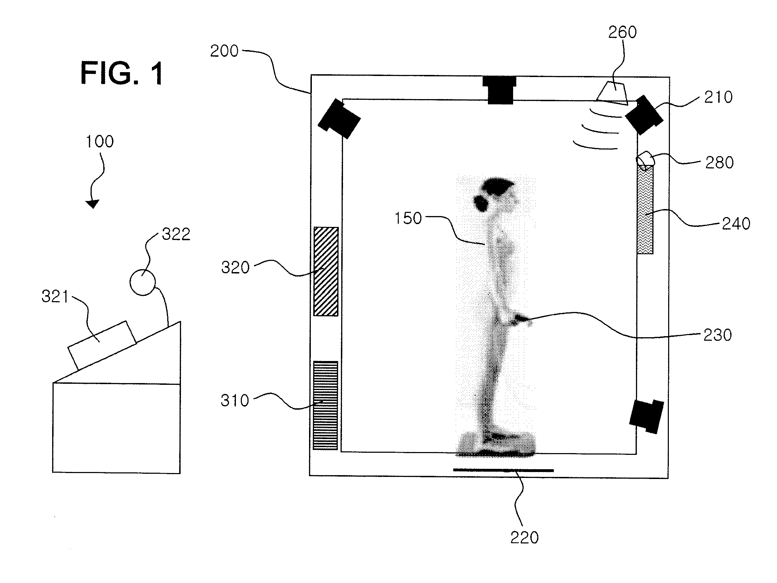

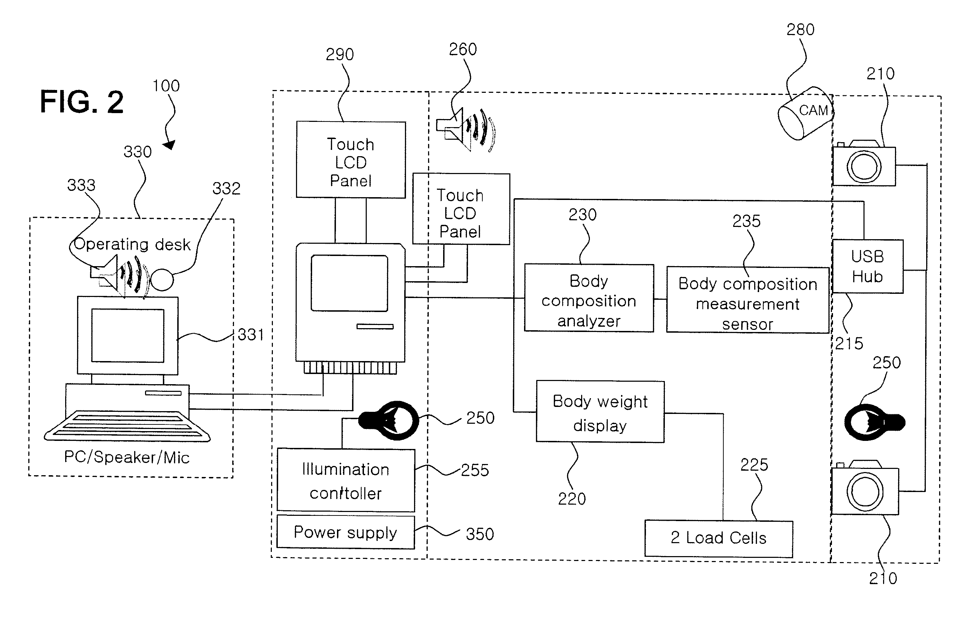

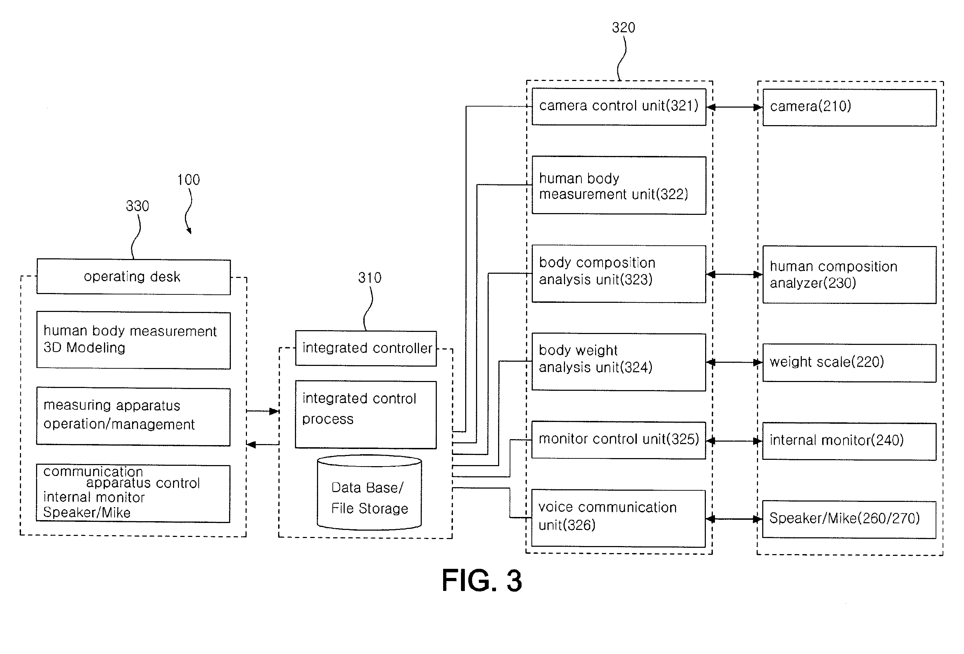

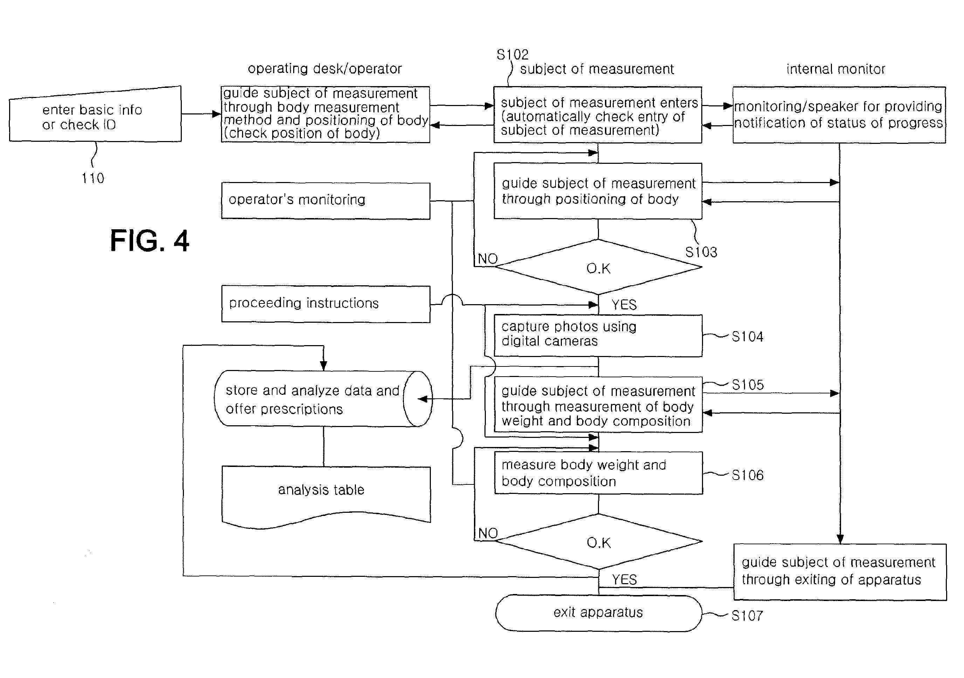

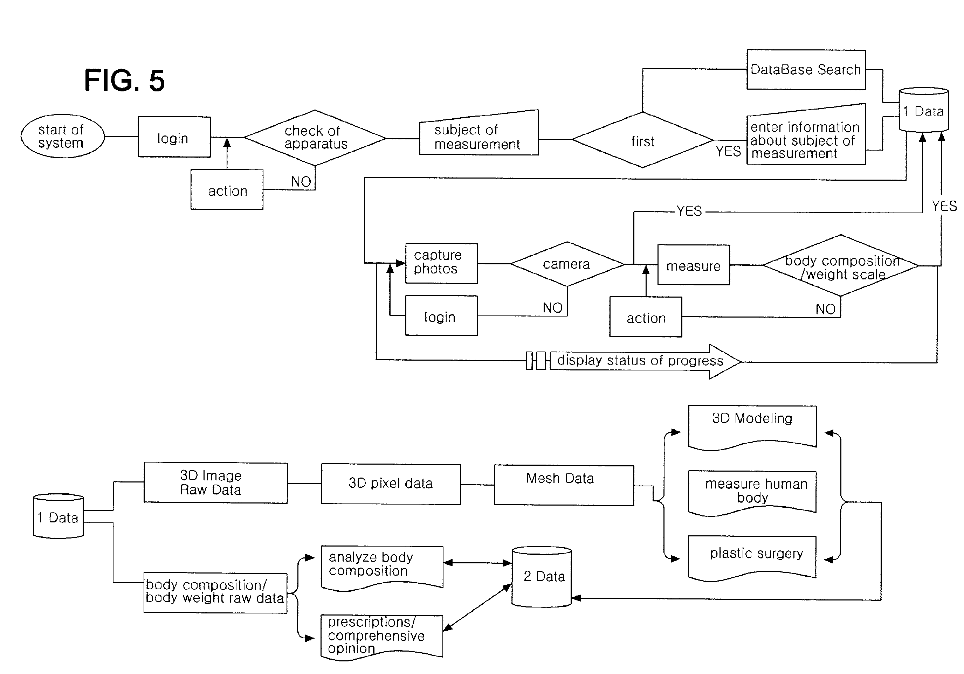

FIG. 1 is a diagram showing the hardware construction of a scan system according to the present invention, FIG. 2 is a block diagram showing the construction of the scan system according to the present invention, FIG. 3 is a diagram showing the software construction of the scan system according to the present invention, FIG. 4 is a block diagram showing the operation of the scan system according to the present invention, and FIG. 5 is a flowchart showing the operation of the scan system according to the present invention.

A 3D image scan apparatus 200 is an apparatus that can be used both in the 3D simulation modeling field for which data about multiview images of a human body and/or an object captured by digital cameras is three-dimensionally converted and used for purposes related to plastic surgery, obesity clinics, health, clothing, shoes, hats and caps, mock-ups, the measurement of dimensions of cultural assets, sculptures, reliefs, medals, trophies, special clothing, gas masks, helmets, glasses, underwear, brassieres and security systems and in the health industry field for which abdominal fat rate, skeletal muscles and basal metabolic amount are analyzed through the measurement of a human body and body weight and the analysis of body composition and then prescriptions are offered.

Here, the engine unit of the software of the scan apparatus converts data measured through the simultaneous photographing of a plurality of digital cameras, that is, a photographing unit 210, into 3D information, and utilizes data acquired by a weight scale for measuring body weight and a body composition analyzer for performing body composition analysis, as follows:

First, the data can be used for the measurement of a human body.

In this case, it is possible to measure every portion of a human body. This information is used in the capacity of information regarding the health of a human body, for example, to assess bending or vertical symmetry. The contour lines of the surface of a human body are used for the analysis of the height or symmetry of a human body or plastic surgery simulation, and are also used for fashion simulation (for example, for clothing, shoes and headgear). Particularly, with regard to plastic surgery, the enlarged or reduced shape of a desired portion of each region of a human body, such as the face, the breast, the abdomen, the inner part of the thigh, the buttocks, and the forearm, is simulated on a 3D screen in real time.

The weight scale measures the weight of a human body, and the body composition analyzer analyzes various types of body composition, such as the body water, protein, inorganic matter, body fat, muscle mass, body fat amount, body fat rate and abdominal obesity of a human body. On the basis of data about the measurement and the analysis, an exercise prescription and a dietary prescription are offered.

Referring to FIGS. 1 to 3, the 3D image scan apparatus 200 having the above-described construction according to the present invention and a 3D image scan system 100 using the 3D image scan apparatus will be described below.

In this case, it is preferred that the outside housing of the scan apparatus 200 be formed of Fiberglass Reinforced Plastic (FRP) or aluminum. However, it will be apparent that the material of the outside housing is not limited thereto and any type of industrially usable plastic and metal may be used as the material of the outside housing.

As shown in the drawings, the apparatus 200 includes at least one photographing unit 210 configured to include a plurality of digital cameras and a hub and send images, captured by the digital cameras, to the Personal Computer (PC) of an integrated controller through the hub, a weight scale 220 configured to measure the body weight of a subject of measurement, and a body composition analyzer 230 configured to measure the body composition of a subject of measurement.

That is, in order to measure the human body and/or body composition characteristics of a subject of measurement 150, the photographing unit 210 including digital cameras is installed on a wall or a ceiling inside the scan apparatus 200. In this case, it is preferred that there be 8 to 16 cameras. In order to enhance precision, a larger number of cameras may be installed when necessary.

The photographing unit 210 generally includes the digital cameras and the hub 215, and performs a function of sending images, captured by the digital cameras, to the PC of the integrated controller 310 through the hub 215.

The photographing unit 210 according to the present invention can acquire 3D modeling data using a plurality of general digital cameras, rather than special cameras. Consequently, there are advantages in that the fabrication of the apparatus and the photographing of images can be facilitated, color photo data can be acquired in real time, and the simulation of the plastic surgery of a desired portion of a human body can be promptly performed using such 3D data.

However, it will be apparent that the photographing unit is not limited to the digital cameras, but that a variety of cameras can be used.

A method of modeling a 3D human body or object according to the present invention which is performed after the human body or object has been photographed using the photographing unit will be described below.

The present invention utilizes multiview image information capable of restoring the 3D shape of an object using the silhouettes and color information of multiview images captured using multiview cameras as a way of capturing shape information from various view points using passive cameras and then restoring the shape and surface color information of an object on the basis of the acquired image information.

In this case, it should be possible to restore a 3D shape model including the geometrical shape and intrinsic surface color texture information of an object and/or a human using the silhouette and color information of multiview images.

Furthermore, this method includes the step of extracting texture coordinates from the results of enlargement of a predetermined central area in a predetermined ratio using the coordinates of a deformed standard model and the results of reduction of a peripheral area in the same ratio with respect to the front image and at least one side image of the face and converting deformed model coordinates into pixel coordinates, and the step of storing the front and side images using the texture coordinates for respective polygons determined by the pixel coordinates and determining the stored results to be deformed textures. In this case, the central area is an area set in the face and around the eyes by a user and the peripheral area is the overall area with the central area of the face excluded.

Accordingly, this method has the advantages of not requiring software or hardware compression and not requiring restoration parts, unlike the conventional technology, creating an excellent texture even using front and side images captured under slightly different illumination conditions, naturally processing the boundaries between polygons, and creating an excellent texture using limited resources, compared to the conventional technology, thus resulting in the creation of a clearer 3D model.

A preferred 3D modeling method thereof is configured as described below.

This method includes the step of receive multiview images obtained by photographing an object from a variety of viewpoints in voxel space composed of voxels of regular size in 3D space including the object and extracting silhouette and color information therefrom, the step of constructing a visual hull through silhouette intersection using the silhouette information, the step of approximating polygons on the section of the visual hull to the intrinsic geometrical shape of the object using the color information while moving along the section, the step of connecting the approximated polygons into a mesh structure and representing the 3D geometrical shape of the object, the step of extracting the color texture of the surface of the object by projecting each mesh structure onto the multiview images, and the step of creating a 3D shape model by modeling the intrinsic shape and surface color information of the object composed of the mesh structure and the color texture corresponding to the mesh structure.

According to this method, when the shape information of a 3D object is restored, it can be expected that the restoration quality of the graph-cut method having restoration accuracy equal to that of a laser scanner can be achieved within the restoration period of the silhouette intersection method that is chiefly used to restore the shape of a dynamic entity.

The restoration of a 3D shape model is performed in 3D space including an object. Respective multiview images of the 3D model have been previously calibrated with respect to the world coordinate system of voxel space. Each image is divided into a foreground including the object and a background.

When an image input unit receives multiview images and transmits the received images to a silhouette and color extraction unit, the silhouette and color extraction unit extracts a silhouette map representing a projected portion of an object to be restored and a color map from the multiview images, and a visual hull restoration unit restores the visual hull in voxel space through silhouette intersection using the extracted silhouette map.

Thereafter, after the polygon approximation process is performed, a mesh structure creation unit creates the overall mesh structure by connecting the polygons of respective sections to each other.

A color texture map having a mesh structure is created by projecting the shape information of an object represented in the mesh structure onto the multiview images, and a 3D shape model creation unit creates a 3D shape model by modeling the intrinsic shape information of the object composed of the mesh structure and the color texture map corresponding to the mesh structure.

A section of the visual hull is composed of linear polygons in which area contour lines are continued, the shape of the actual object is included within the polygons, and corresponding points, such as colored corresponding lines 150, 151 and 152, can be obtained along polygon lines obtained by projecting respective polygon segments onto the multiview images.

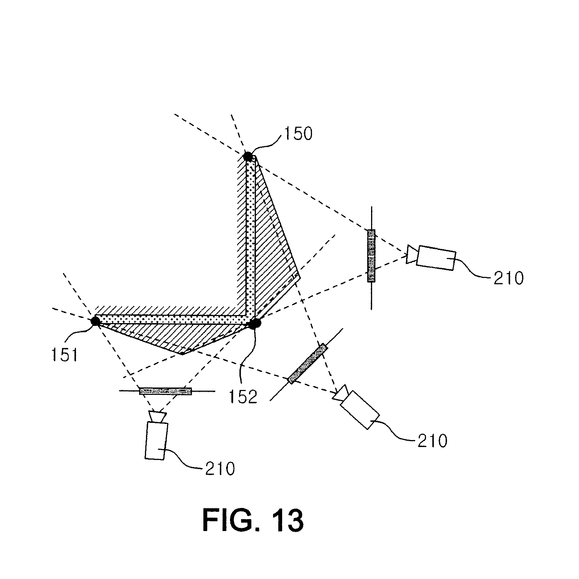

The 3D shape represented in the overall mesh structure created by the polygon approximation process is **characterized in that** it is projected onto all pixels corresponding to an object silhouette area when projected onto the multiview images, and the edge between adjacent meshes of the mesh structure is **characterized in that** it is developed along the color edge component of the surface of the object. That is, as shown in FIG. 13, the shape of an object is desirably represented and the silhouette maps of respective images are all satisfied.

Next, the constructions of the weight scale and the body composition analyzer will be described.

The weight scale 220 for measuring a subject of measurement 150 is provided on the bottom of the scan apparatus 200 on which the subject of measurement 150 is located, and the body composition analyzer 230 for measuring body composition is connected to a system control unit 320 and an external PC 331 and sends measured data.

Accordingly, the body weight and body composition of the subject of measurement 150 can be simultaneously measured and/or analyzed at the same location, so that customized exercise and dietary prescriptions can be presented based on a variety of types of body composition analysis data, such as data about the body water, protein, inorganic matter, body fat, muscle mass, amount of body fat, body fat rate, abdominal obesity of a human body.

The body composition analyzer 230 generally uses a bioelectrical impedance method. The body composition analyzer 230 includes a plurality of electrodes configured to come into contact with the hands of the subject of measurement 150 and input and output means configured to include an input unit for inputting information about the subject of measurement 150, such as gender, age and human body measurement data, and a display unit for displaying measured states and measurement results.

In this case, in order to increase the accuracy and diversity of examination, it is preferable to attach a sensor 235 onto the top of the weight scale 220 on which the feet of the subject of measurement 150 are located and obtain data through the hands and feet of the subject of measurement 150.

Furthermore, a plurality of illuminators 250 for aiding taking of photos is installed on a wall or a ceiling, in which case the plurality of illuminators 250 is connected to an illumination controller 255.

A speaker 260 is communication means by which an operator at an operating desk 330 outside the scan apparatus transmits information and instructions to the subject of measurement 150 through an operator microphone 332.

Meanwhile, an internal monitor 240 is installed inside the scan apparatus 200 so that the subject of measurement 150 can monitor all types of status, and a CAM 280 may be installed inside the scan apparatus 200 so that the operator outside the scan apparatus 200 can easily view the location and motion of the subject of measurement 150 from the outside.

Furthermore, a power supply 350 for supplying power to the various types of photographing, measuring and illumination apparatuses is provided.

Furthermore, a system control unit 320 is connected to the external PC 331 or provided inside the wall of the scan apparatus 200, and performs the functions of testing and maintaining the scan system 100 and controls the photographing unit 210, the weight scale 220, the body composition analyzer 230, the internal monitor 240, the illuminators 250, the speaker 260 and the microphone 270 through the PC 331.

The system control unit 320 includes a camera control unit 321 for controlling the calibration and photographing of the photographing unit 210; a human body measuring unit 322 for performing 3D modeling using a measuring technique based on contour lines, colors and squares; a body composition analysis unit 323 for analyzing body composition and offering prescriptions on the basis of data measured by the body composition analyzer 230; a body weight analysis unit 324 for analyzing and managing body weight on the basis of data measured by the weight scale 220; a monitor control unit 325 for controlling the internal monitor 240; and a voice communication unit 326 for managing the speaker 260 and the microphone 270.

The internal monitor 240 is provided for the subject of measurement 150 located inside the scan apparatus 200, and the speaker 260 and microphone 270 for providing information about the status of progress and the correction of a position is used to support communication between the subject of measurement inside the scan apparatus 200 and the operator. Meanwhile, it is preferable to use a camera SDK so as to control the cameras.

The human body measurement system according to the present invention used in the method of measuring a human body will be described below.

The integrated controller 310 is further provided to analyze and store data captured and measured inside the scan apparatus 200 and images, body weight data and body composition data acquired through the system control unit 320. The integrated controller 310 includes an integrated control process 311 for storing a program in charge of integrated control and a database/file storage unit 312.

In general, the integrated controller 310 is implemented in the form of a microcomputer. That is, the integrated controller 310 is a processor formed of a microcomputer and configured to centrally control the body composition analyzer 230, and is used to store a predetermined operation program in embedded memory and perform sequential input and output control according to the running of the program.

An electrode current application and voltage detection unit functions to supply AC sine wave current in a predetermined sequence, receive detected voltages from relevant electrodes, calculate the impedance (body fat etc.) of a human body, receive the phase difference and magnitude signals of the voltage and current from a phase comparison unit, analyze corresponding body composition, and send analysis result data to the operator PC through an interface.

An operating desk 330 is provided outside the scan apparatus 200 to connect to the integrated controller 310 over a network and remotely manage and operate the photographing unit 210 and the measuring devices 220 and 230. It is preferred that the operating desk 330 include the external PC 331, the speaker 332 and the microphone 333 for performing 3D modeling for the measurement of a human body, the operation and management of the measuring devices, and the control of the communication device.

Furthermore, as shown in FIG. 2, a touch LCD Panel 290 is provided on a wall of the scan apparatus to enable wireless communication to be made with the external PC, and the body composition analyzer 230 is generally provided with a body composition measuring sensor 235. Furthermore, a 2 Load cell 225, that is, the weight scale, is connected to a body weight display 220.

The human body measurement system 100 using the 3D image scan apparatus 200 constructed as described above may be used in the following applications.

A first application system is hardware, and may include the digital camera 210, the weight scale 220, and the body composition analyzer 230. This first application system may be easily used in plastic surgery for all portions of the human body of a subject of measurement, such as the face, the breast, the abdomen, the inner part of a thigh and the forearm, using human body measurement and body composition analysis.

A second application system is hardware, and may include the digital camera 210, the weight scale 220, and the body composition analyzer 230. This second application system may be used to provide appropriate exercise and dietary prescriptions to a subject of measurement based on human body measurement and body composition analysis.

A third application system is hardware, and may include the digital camera 210, the weight scale 220, the body composition analyzer 230, a sphygmomanometer, a glucometer and an X-ray machine. This third application system can be used to provide exercise and dietary prescriptions through the human body measurement and body composition analysis of a subject of measurement.

A face-dedicated plastic surgery simulation system is hardware, and may include the digital camera 210 and an X-ray machine (an optional element). This face-dedicated plastic surgery simulation system may be used in the manufacture of special shoes, helmets, glasses, gas masks and special hats or the manufacture of military customized shoes, and may be also used in plastic surgery for the removal of scars, cosmetic surgery and human body plastic surgery.

A clothes simulation system is hardware, and may include a full-length digital camera 210. This clothes simulation system may be used in the customized manufacture of special clothes and ready-made clothes.

Furthermore, an industrial modeling process may be applied to avatars, 3D games, actual length manufacture, the manufacture of models, molds and mock-ups, security, medical service, archaeology, anthropology, criminology, etc.

It will be apparent that although not described, the present invention can be applied in various industry fields in which captured images must be three-dimensionally modeled.

A 3D image scan method using the scan system 100 according to the present invention will be described below with reference to FIGS. 4 and 5.

First, the operator performs a login step S101 of entering the basic information of a subject of measurement 150 and undergoing ID authentication. Thereafter, there are performed an automatic entry checking step S102 of the operator at the operating desk 330 checking whether the subject of measurement 150 has entered the scan apparatus 200 after guiding the subject of measurement 150 through the measurement method to be used, a step S103 of the operator guiding the a subject of measurement 150 through the appropriate position of a human body using the internal monitor 240 and the speaker 260, a step of the process returning to the guiding step if the body position of the subject of measurement 150 is inappropriate or proceeding to a step S104 of performing photographing using one or more internal photographing devices 210 if the position of the human body is appropriate, a step S105 of guiding the subject of measurement 150 through a process of measuring the body weight and body composition through the internal monitor 240, a step S106 of measuring the body weight and body composition of the subject of measurement 150 using the weight scale 220 and the body composition analyzer 230, a step S107 of the subject of measurement 150 exiting the scan apparatus 200 after the measurement has been completed, a step S108 of storing measured data about the subject of measurement data in the integrated controller, analyzing the measured data, and providing prescriptions, and an analysis table output step S109 of outputting analysis and prescription data.

Here, it is preferred that step S108 include the step of storing raw data of images of the subject of measurement acquired by the cameras in a DB in the form of data about the subject of measurement regarding 3D modeling, human body measurement and plastic surgery via voxel data (3D pixel data) and mesh data, and storing data about the analysis of the body composition analysis, the prescriptions and a comprehensive opinion based on the raw data of the body composition and the body weight in the DB as well.

The operating desk 330 takes charge of the progress of a procedure until the subject of measurement 150 exits in such a way that from there, real-time guidance is provided, instructions as to how to proceed are given and the subject of measurement 150 is monitored.

Next, the human body measurement function of the present invention which can be used in the human body measurement system will be described in detail below.

First, a function of measuring portions of a human body and representing them in a 3D shape in the form of numerical values and a function of representing numerical values in a 3D shape can be performed. Furthermore, a function of performing 3D comparison using standard bodily shape data, a function of expanding/editing and measuring a freely designated portion of measurement and a function of adjusting the location of a portion of measurement can be performed. Furthermore, a function of representing a human body using contour lines and colors and a function of performing measurement while drawing a line on a background in the case of comparison or laterally bending a contour line can be performed.

Furthermore, a function of representing a freely designated portion in the form of a section, a function of representing measured values in a 3D shape on the basis of the measurement of body fat, a function of creating a standard model based on the height using standard Mesh data, a function of interfacing 2D and 3D programs with each other, and a function of using basic skeleton completion technology can be performed. In this case, view types displayed on a screen include mesh, texture and contour types.

Furthermore, when the body composition and the body weight are measured, 3D representation can be performed. In this case, it is preferable to represent the comparisons between measured values for respective periods.

It is preferable to set a simulation program so that in the case of simulation, it is possible to deform each portion of the human body of a subject of measurement and perform the comparison with a standard bodily shape, and in the case of dietary prescription, it is possible to create graphs and characters and insert various types of content, thereby enabling 3D representation.

In the field of 3D shapes, the system is configured to perform a calibration function and a 3D image creation function when capturing photos.

In more detail, in the field of human body measurement, it is possible to correct the height, the sitting height, the outside and inside lengths of the right and left lower parts of a human body, the outside and inside lengths of the arms, and the height of a subject of measurement attributable to the opening of the legs based on the lengths of the right and left feet and measure any portions of a human body as well as the shoulder, the head, the neck, the upper arm, the breast, the waist, the buttocks, the inner part of the thigh and the calf of the leg.

In the field of plastic surgery, it is preferable to enable the insertion of a prosthesis into a 3D shape by using a database or freely designating a volume, the change of a 3D shape by using a mouse and a touch screen, and the removal of a specific portion of a 3D shape using zooming in and out, cc designation and free cutting.

In the analysis of body composition, for the whole body, intracellular fluid, extracellular fluid, protein, inorganic matter and body fat are used, and for body portions, (the trunk, the left arm, the right arm, the left leg and the right leg) x body fat, body water and muscle mass is used. In the measurement of the body weight, it is preferable to separately measure and analyze left and right body weights using left and right body weight analysis and 3D modeling techniques. In this case, it is more preferable to perform implementation so as to maintain a body weight accuracy of +/- 50g.

In order to compensate for the analysis of body composition data, the body weight, the muscle mass, the amount of body fat, the body fat rate and the extracellular fluid ratio are considered and analyzed for the muscle and body fat balance, the correlation between the body weight, the body fat, the abdominal fat rate and muscle is considered and analyzed for the assessment of the bodily shape, the abdominal fat rate, the hypodermic fat area, the visceral fat area, the abdominal fat area and VSR are considered and analyzed for the analysis of abdominal obesity, BMI, WHI, WHR, BMR, Kcal and the extracellular fluid ratio are considered and analyzed for the provision of reference values, and the body water, protein, inorganic matter and body fat (represented in a Pie chart) are considered and analyzed for the analysis of body composition.

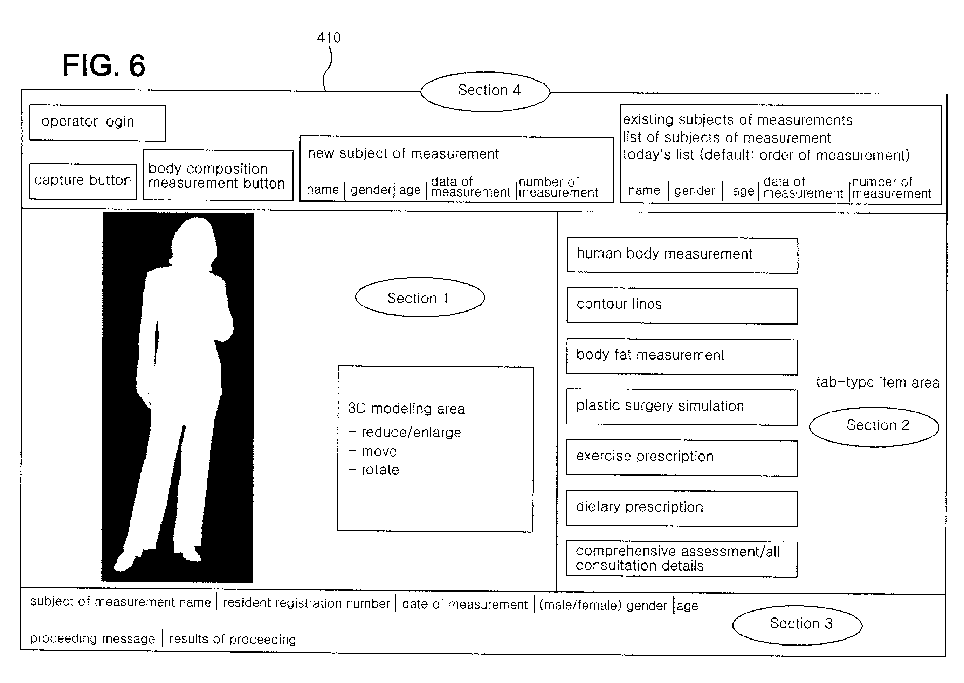

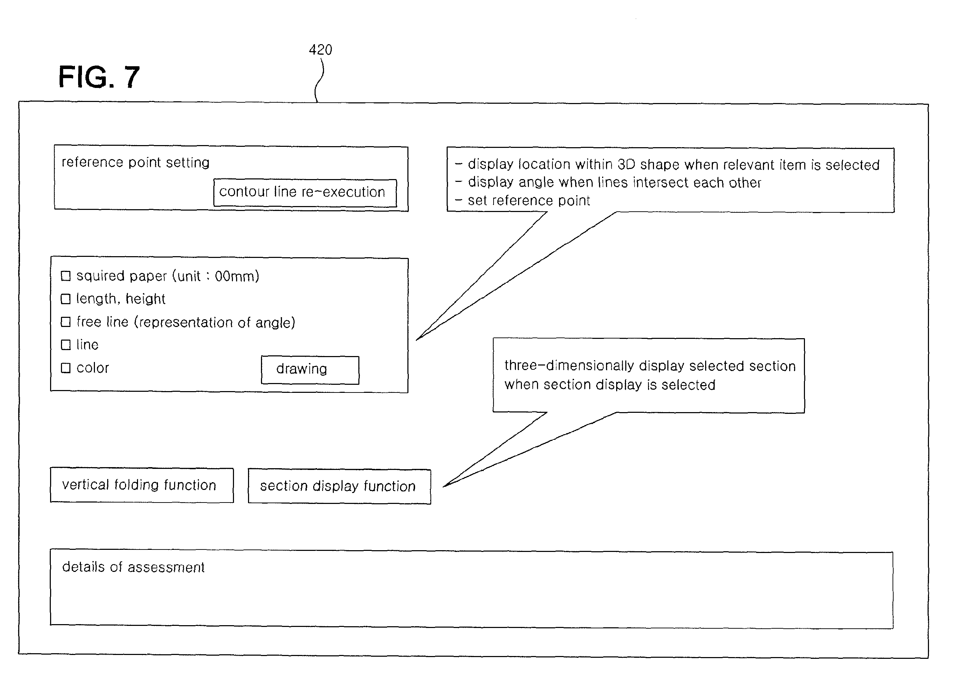

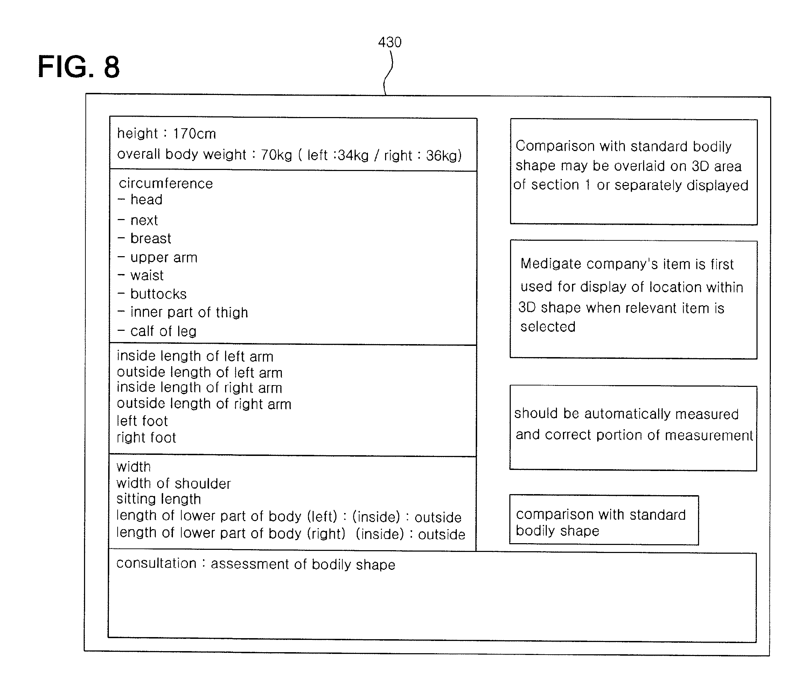

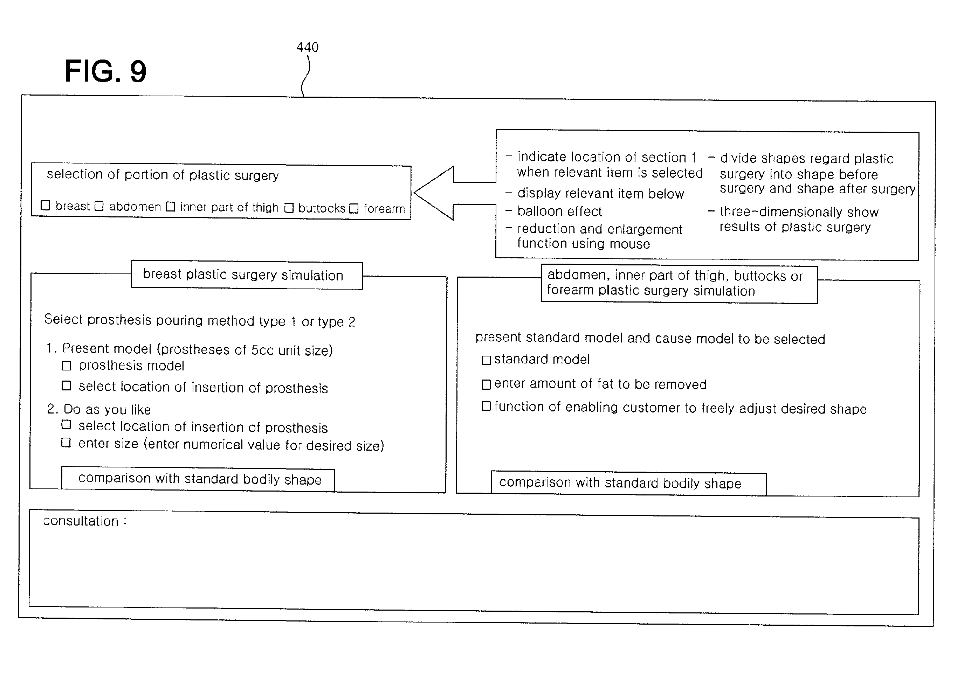

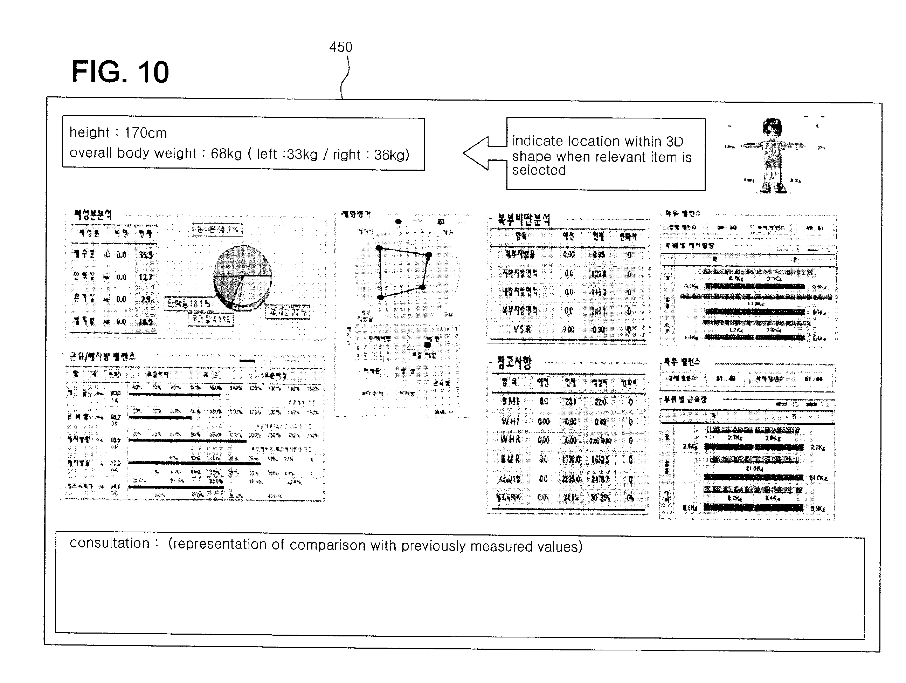

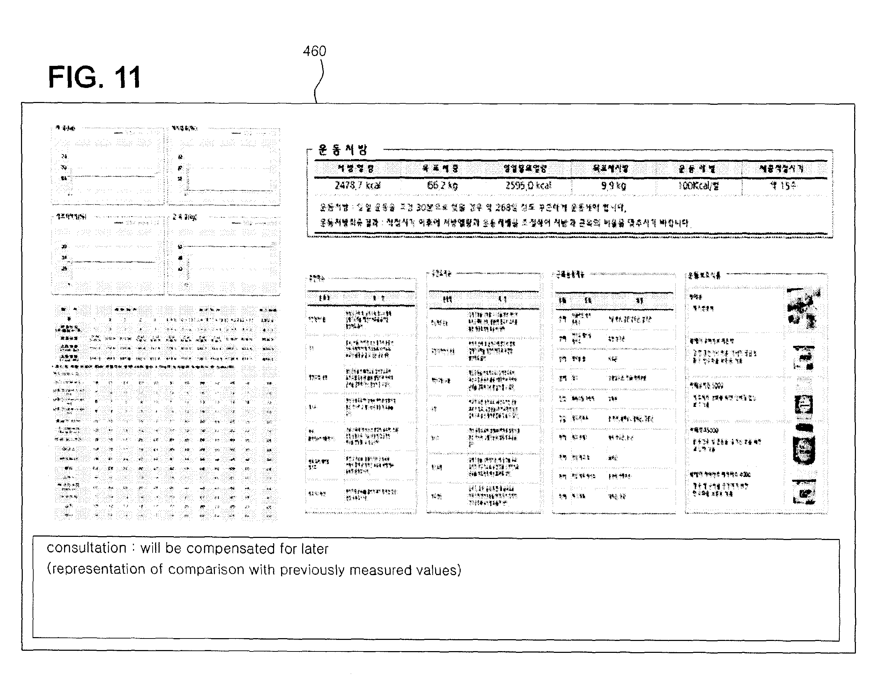

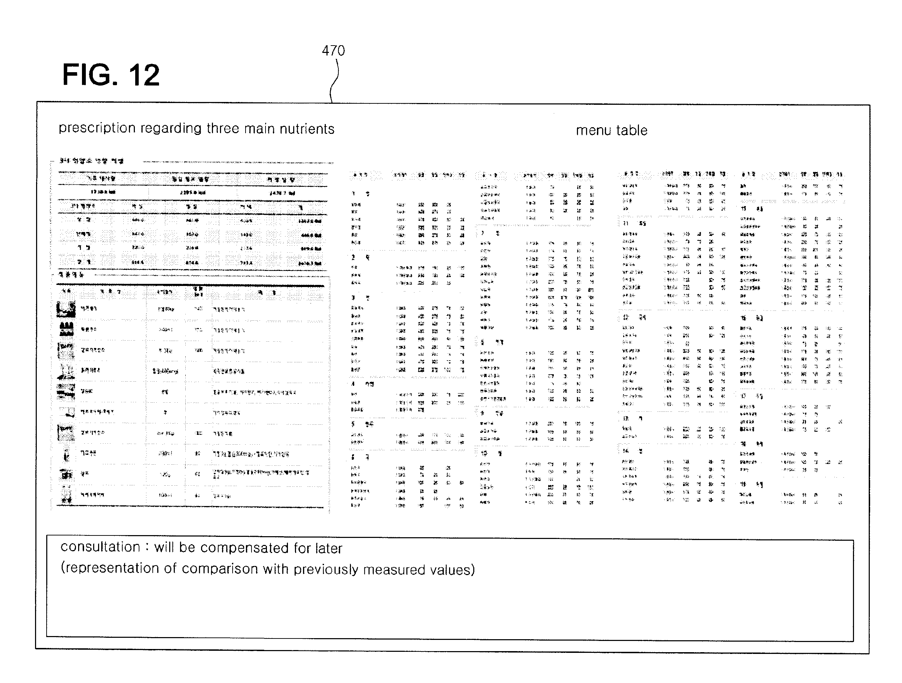

A preferred embodiment of a 3D image scan system output screen according to the present invention will be described below. FIG. 6 is a diagram showing the construction of a main output screen of the scan system according to the present invention, FIG. 7 is a diagram showing the construction of a contour line screen of the scan system according to the present invention, FIG. 8 is a diagram showing the construction of a human body measurement screen of the scan system according to the present invention, FIG. 9 is a diagram showing the construction of a simulation input screen of the scan system according to the present invention, FIG. 10 is a diagram showing the construction of a human body measurement screen of the scan system according to the present invention, FIG. 11 is a diagram showing the construction of an exercise prescription screen of the scan system according to the present invention, and FIG. 12 is a diagram showing the construction of a dietary prescription screen of the scan system according to the present invention. As will be apparent from the illustrated drawings and screen configurations, the human body measurement system according to the present invention can construct the screens of FIGS. 6 to 12 through photographing of single round and the analysis and measurement of the body composition analyzer.

First, referring to FIG. 6, the configuration of a main screen 410 will be described. The upper section 4 of the main screen 410 is configured such that operator login can be performed, a photographing button and a body composition measurement button are provided, the name, gender, age, date of measurement and number of measurements of a new subject of measurement can be entered, and, preferably, a list of existing subjects of measurement, a current day's list (default: in the sequence of measurement), names, genders, ages, dates of measurement and the numbers of measurements can be entered.

Section 1 on a side of the middle portion of the screen is configured such that a 3D modeling area is set and an optimized screen is provided based on captured photos through reduction or enlargement, movement and rotation.

Section 2, which is located on the right side of the screen and is a tab-type item area, is configured such that tab keys related to the measurement of a human body, contour lines, the measurement of body fat, the simulation of plastic surgery, an exercise prescription, a dietary prescription, comprehensive assessment and details of the consultation so that editing and management can be facilitated.

The screen is set so that editing and management are facilitated.

Section 3 on the lower portion of the screen is configured such that the name, resident registration number, date of measurement, gender (male/female), age, proceeding message, proceeding results and status of a subject of measurement can be displayed.

That is, section 1, section 2, section 3 and section 4 are configured to be a 3D area, an item area, a message area and a login and basic data entry area, respectively. It is preferred that the screen be configured such that when each item is selected from the item area, switching to the 3D area or the message area can be freely performed.

Next, referring to FIG. 7, a contour line screen 420 will be described. This screen is used to represent contour lines in the 3D area of section 1. In this screen, a reference point is set by the re-execution of contour lines, and a drawing section composed of squared paper (that is, a form in which a unit can be freely adjusted), length/height, free line (angle representation), line and color items is provided. Here, the screen is configured such that when relevant items are selected, functions of indicating a location within a 3D shape, indicating an angle formed when lines intersect each other, and setting a reference point can be performed. Furthermore, the screen is configured to enable a vertical folding function and a section display function, and a selected section is three-dimensionally displayed by the selection of section display. Moreover, a section for making comments on the results of assessment is set in the lower portion of the screen.

As shown in FIG. 8, the measured values of a human body are displayed on a human body measurement screen 430. As shown in this drawing, the height and the overall body weight are displayed on the upper portion of the screen. In this case, it is preferred that the body weight be displayed for right and left portions.

Data about the head, the neck, the breast, the forearm, the waist, the buttocks, the inner part of the thigh and the calf of the leg is entered as circumference indications, and the inside length of the left arm, the outside length of the left arm, the inside length of the right arm, the outside length of the right arm, the left leg, the right leg, the width of the shoulder, the sitting height, the length of the left lower part of the human body : the inside length of the left lower part : the outside length of the left lower part, and the length of the right lower part of the human body : the inside length of the right lower part : the outside length of the right lower part are entered.

In this case, the comparison with a standard bodily shape may be overlaid on the 3D area of section 1 or separately indicated, should be automatically measured, and should be able to correct a portion of measurement. Furthermore, a consultation section regarding the assessment of the bodily shape is set in the lower portion of the screen.

Next, referring to FIG. 9, a simulation input screen 440 used to enter values for plastic surgery will be described.

First, on a side of the upper portion of the screen, a target portion for plastic surgery can be selected from among the breast, the abdomen, the inner part of the thigh, the buttocks and the forearm. In this case, when an item is selected, the location of section 1 is indicated, the corresponding item is indicated in a portion below the upper portion, and a balloon effect and a reduction and enlargement function using a mouse may be added. Furthermore, shapes regarding plastic surgery are divided into a shape before surgery and a shape after surgery, and the results of the plastic surgery will be shown three-dimensionally.

As an example, in a breast plastic surgery simulation screen, a prosthesis insertion method is selected by selecting No. 1 or No. 2, a model (prostheses of 5cc size) is presented, and a prosthesis model and the location of insertion of a prosthesis are allowed to be selected, or the selection may be freely performed by a user.

In a plastic surgery simulation related to the abdomen, the inner part of the thigh, the buttocks or the forearm, a standard model is presented and allowed to be selected, the amount of fat to be removed is allowed to be entered, and a desired shape is allowed to be adjusted by a customer. In this case, it is preferable to configure a screen so that a separate consultation section is provided in the lower portion of the screen, thereby facilitating management.

As shown in FIG. 10, the data and graph regarding the analysis of the body composition analysis of the subject of measurement 150, data about muscle and body fat balance, a bodily shape assessment item, the analysis of abdominal obesity, right and left balance and the muscle mass for each body portion are indicated on a human body measurement screen 450 for displaying the measured values of a human body. A consultation section is provided in the lower portion of the screen to allow comparison with previously measured values.

As shown in FIG. 11, in an exercise prescription screen 460, the body weight, the body fat and the muscle mass are indicated, a corresponding exercise prescription is presented, and information about a recommended exercise menu, an aerobics menu, a muscle exercise menu and auxiliary food is provided in a customized manner. A consultation section is provided in the lower portion of the screen to allow comparison with previously measured values.

As shown in FIG. 12, a three major nutrient prescription, a food menu and a menu table are provided in a dietary prescription screen 470 in a customized manner. A consultation section is provided in the lower portion of the screen to allow comparison with previously measured values.

It will be apparent that the above-described basic output screens (FIGS. 6 to 12) are merely examples and the output screens may be configured in different manners.

As described above, according to the scan system of the present invention, the photographing unit is constructed of a plurality of general digital cameras, the digital cameras are installed at various angles inside the scan apparatus and operated to capture photos at the same time, and 3D data can be extracted using multiview images, so that there are advantages in that processing speed is increased and a variety of types of 3D modeling editing can be performed.

Furthermore, the measurement of the body composition and human body of a subject of measurement can be performed at the same time, so that the uncertainty of data provided by a subject of measurement can be eliminated, thereby improving the accuracy of measurement and analysis results, and photographing and measurement can be performed at the same location, so that the measurement of a human body and the analysis of body composition can be performed conveniently and rapidly, thereby facilitating multipurpose use.

Although the preferred embodiments of the present invention have been disclosed for illustrative purposes, those skilled in the art will appreciate that various modifications, additions and substitutions are possible, without departing from the scope and spirit of the invention as disclosed in the accompanying claims.

## Claims

1. A human body measurement system into which a human body measurement apparatus and a measurement unit are incorporated, comprising:
a photographing unit configured to comprise a plurality of cameras and a hub and send images, captured by the cameras, to a Personal Computer (PC) of an integrated controller through the hub;
a weight scale configured to measure body weight of a subject of measurement; and
a body composition analyzer configured to measure body composition of the subject of measurement.

2. The human body measurement system as set forth in claim 1, wherein the image data captured by the photographing unit is three-dimensionally modeled to provide measurement of length and volume of each portion of a human body, 3D simulation is provided based on the measured data, and information about exercise and dietary prescriptions is provided on the basis of the measurement of the body weight using the weight scale and the analysis of the body composition using the body composition analyzer;
wherein the simulation and the information are provided simultaneously.

3. The human body measurement system as set forth in claim 1, wherein the body weight is measured using the weight scale at the same time that photographing is performed using the photographing unit, height is measured using the captured data, and the measured data is automatically input to the body composition analyzer and used for analysis of the body composition data measured by the body composition analyzer.

4. The human body measurement system as set forth in claim 3, wherein a 3D shape model is formed by approximating shape information using polygons through extraction of colored corresponding points of multiview images captured by the photographing unit, connecting polygons of each section to each other and then creating a complete mesh structure.

5. The human body measurement system as set forth in claim 1, further comprising:
a system control unit for testing and maintaining the system and controlling the photographing unit, the weight scale, the body composition analyzer, an internal monitor, an illuminator, a speaker and a microphone through a Personal Computer (PC);
an integrated controller for analyzing and storing acquired images, body weight data and body composition data through the system control unit;
an operating desk for connecting to the integrated controller over a network and remotely managing and operating the cameras and the analyzer; and
a power supply for supplying power to the system.

6. The human body measurement system as set forth in claim 5, wherein the measurement apparatus further comprises an internal monitor for enabling the subject of measurement to monitor status of progress.

7. The human body measurement system as set forth in claim 5, wherein the operating desk comprises a Personal Computer (PC) for referring to and managing measured data, performing simulation analysis and offering prescriptions, and a communication microphone and speaker for transmitting information about a position of measurement and status of progress to the subject of measurement.

8. The human body measurement system as set forth in claim 5, wherein the system control unit comprises:
a camera control unit for controlling calibration and photographing processes;
a human body measuring unit for performing 3D modeling and performing measurement based on captured images of a human body of the subject of measurement;
a body composition analysis unit for analyzing body composition and offering prescriptions on the basis of data measured by the body composition analysis unit;
a body weight analysis unit for analyzing and managing body weight based on data measured by the weight scale;
a monitor control unit for controlling the internal monitor; and
a voice communication unit for managing the speaker and the microphone.

9. An information provision method using the human body measurement system set forth in any one of claims 1 to 8, comprising the steps of:
entering basic information of a subject of measurement and performing ID authentication login;
an operator at the operating desk guiding the subject of measurement through a measurement method and checking whether the subject of measurement has entered the scan apparatus;
the operator guiding the subject of measurement through positioning of a human body;
returning to the guiding step if a position of the human body of the subject of measurement is inappropriate, and performing photographing using the cameras if a position of the human body is appropriate;
guiding the subject of measurement through a procedure of measuring body weight and body composition through the internal monitor;
measuring the body weight and body composition of the subject of measurement using a weight scale and a body composition analyzer;
after the measurement, the subject of measurement exiting the scan apparatus; and
storing measured data about the subject of measurement in the integrated controller, analyzing the measured data, and offering prescriptions.

10. The information provision method as set forth in claim 9, wherein the step of storing measured data, analyzing the measured data and offering prescriptions comprises the step of storing raw data of images of the subject of measurement acquired by the cameras in a DB in the form of data about the subject of measurement regarding 3D modeling, human body measurement and plastic surgery via voxel data (3D pixel data) and mesh data, and storing data about the analysis of the body composition analysis, the prescriptions and a comprehensive opinion based on the raw data of the body composition and the body weight in the DB as well.

11. The information provision method as set forth in claim 9, further comprising, after the step of storing measured data, analyzing the measured data and offering prescriptions, the step of outputting data about the analysis and the prescription.
